# EUROPÄISCHE PATENTANMELDUNG

(11) **EP 2 623 090 A1**
(43) Veröffentlichungstag der Anmeldung: **07.08.2013**
(21) Anmeldenummer: 12187565.2
(22) Anmeldetag: 08.10.2012
(51) Int. Cl.: A61K 8/36, A61K 8/368, A61K 8/49

(54) **Biofilmreduzierende Spezialzahncreme**

(30) Priorität: 28.10.2011 DE 102011085366
(71) Anmelder: Henkel AG&Co. KGAA, 40589 Düsseldorf (DE)
(72) Erfinder: Miehlich, Kristin, 42119 Wuppertal (DE); Veith, Birgit, 40235 Düsseldorf (DE); Boy, Julia, 45479 Mülheim an der Ruhr (DE); Simmering, Rainer, 41515 Grevenbroich (DE); Breves, Roland, 40822 Mettmann (DE); Zelic, Daniela, 40883 Ratingen (DE)

(57) **Zusammenfassung**

Besonders plaquereduzierende Mund- und Zahnpflege- und -reinigungsmittel, enthalten - bezogen auf ihr Gewicht - 0,01 bis 1 Gew.-% mindestens einer D-Aminosäure und 0,1 bis 5 Gew.-% mindestens eines Antiplaque-Wirkstoffes aus den Gruppen der Alkypyridiniumsalze und/oder der Biguanide und/oder der Salze von Organo-polyphosphonsäuren und/oder p-Hydroxybenzoesäuremethyl-, -ethyl- oder propylester und/oder Natriumsorbat und/oder Natriumbenzoat.

## Beschreibung

Die Erfindung betrifft eine Zahncreme bzw. Zahnpasta, die den besonderen Erfordernissen bei der Entfernung von Biofilmen (Plaque) Rechnung trägt.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Neben der Reinigung der Zähne erwartet der Verbraucher von den gattungsgemäßen Produkten auch eine Pflege der Zähne und der Mundhöhle. So sind insbesondere ein "sauberes" Gefühl, d.h. eine glatte und glänzende Zahnoberfläche sowie ein frisches Gefühl im Mund wesentliche Aspekte für den Kaufanreiz von Zubereitungen zur Mund- und Zahnpflege- und -reinigung. Ein erfolgreiches Mittel der gattungsgemäßen Art sollte daher die Zähne gründlich reinigen, ohne den Zahn oder die Zahnoberfläche zu schädigen und gleichzeitig Mundgeruch verringern und/oder verhindern.

In unzugänglichen Bereichen des Mundes wie beispielsweise in Zahnzwischenräumen oder die den hinteren Backenzähnen ist die herkömmliche mechanische Reinigung mit der Zahnbürste oft nicht vollständig erfolgreich, insbesondere dann, wenn die Putzdauer nicht ausreichend ist. Dies hat zur Folge, daß der Zahnbelag in diesen Bereichen nicht vollständig entfernt wird und sich Mikroorganismen wieder auf den Zähnen ansiedeln können, was zur Plaqueneubildung führt.

Es ist bekannt, Biofilme auf verschiedensten Oberflächen durch D-Aminosäuren abzubauen. Hierzu schlägt die WO 2011/085326 A1 vor, eine geeignete Menge einer D-Aminosäure auf ein biofilmbelastetes Objekt zu applizieren. Der Schwerpunkt liegt dabei auf Implantaten; Körperoberflächen allgemein oder die Mundhöhle im Besonderen werden nicht explizit adressiert.

Es besteht daher nach wie vor das Bedürfnis, Plaque zu reduzieren, Halitosis zu bekämpfen und Wirkstoffe oder Wirkstoffkombinationen dafür bereitzustellen, die frei von den genannten Nachteilen sind.

Der vorliegenden Erfindung lag daher die Aufgabe zugrunde, ein Mund- und Zahnpflege- und -reinigungsmittel bereitzustellen, das besonders plaquereduzierend wirkt und die vorstehend genannten Nachteile überwindet. Insbesondere sollte eine hohe Reinigungsleistung und Plaqueentfernung erzielt werden.

Es wurde nun gefunden, daß die Kombination bestimmter Antiplaque-Wirkstoffe mit D-Aminosäuren die vorstehend genannten Aufgaben löst. Insbesondere einige ausgewählte Antiplaque-Wirkstoffe zeigen in Kombination mit D-Aminosäuren deutliche Effekte, die über die Wirkung der Einzelsubstanzen weit hinausgehen.

Gegenstand der vorliegenden Erfindung ist in einer ersten Ausführungsform ein Mund- und Zahnpflege- und -reinigungsmittel, enthaltend - bezogen auf sein Gewicht-
a) 0,01 bis 1 Gew.-% mindestens einer D-Aminosäure,
b) 0,1 bis 5 Gew.-% mindestens eines Antiplaque-Wirkstoffes aus den Gruppen
   a. der Alkypyridiniumsalze und/oder
   b. der Biguanide und/oder
   c. der Salze von Organo-polyphosphonsäuren und/oder
   d. p-Hydroxybenzoesäuremethyl-, -ethyl- oder propylester und/oder
   e. Natriumsorbat und/oder
   f. Natriumbenzoat.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes, Mund- und Zahnspülungen sowie Mund- und Zahngele. Bevorzugt geeignet sind Zahnpasten und flüssige Zahnreinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden. Eine weitere besonders bevorzugte Ausführungsform der vorliegenden Erfindung sind Mundspüllösungen und Mundwasser, die zum Ausspülen der Mundhöhle verwendet werden.

Als ersten wesentlichen Inhaltsstoff enthalten die erfindungsgemäßen Mittel 0,01 bis 1 Gew.-% mindestens einer D-Aminosäure.

Imn Rahmen der vorliegenden Erfindung besonders geeignete D-Aminosäuren sind (mit ihren CAS-Nummern): D-Alanin (338-69-2), D-Asparagin Monohydrat (3130-87-8), D-Asparaginsäure (1783-96-6), D-Serin (312-84-5), D-Phenylalanin (673-06-3), D-Leucin (328-38-1), D-Tryptophan (153-94-6), D-Histidin (351-50-8), D-Ornithin Monohydrochlorid (16682-12-5), D-Valin (640-68-6), D-Prolin (344-25-2), D-Glutamin (5959-95-5), D-Arginin (157-06-2), D-Methionin (348-67-4), D-Glutaminsäure (6893-26-1), D-Arginin Monohydrochlorid (627-75-8), D-Histidin Monohydrochlorid Monohydrat (6341-24-9), D-Lysin Monohydrochlorid (7274-88-6), D-Tyrosin (556-02-5).

Einige der vorstehend genannten D-Aminosäuren steigern allein oder in Mischung miteinander die Wirkung des/der zweiten wesentlichen Inhaltsstoffe(s) besonders deutlich. So sind erfindungsgemäß besonders bevorzugt Mund- und Zahnpflege- und -reinigungsmittel **dadurch gekennzeichnet, daß** sie 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin enthalten.

Auch D-Tyrosin ist besonders geeignet, so daß weitere bevorzugte Mund und Zahnpflege- und - reinigungsmittel 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin enthalten.

Eine weitere besonders geeignete -Aminosäure ist das D-Metionin. Hier sind erfindungsgemäße Mund und Zahnpflege- und -reinigungsmittel bevorzugt, die 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin enthalten.

Auch D-Tryptophan ist besonders geeignet, so daß weitere bevorzugte Mund und Zahnpflege- und -reinigungsmittel 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan enthalten.

Ganz besonders wirksam sind Mischungen aus D-Leucin und D-Tyrosin, weswegen Mund und Zahnpflege- und -reinigungsmittel, die sowohl D-Leucin als auch D-Tyrosin enthalten, bevorzugt sind.

Auch die Dreierkombinationen D-Leucin und D-Tyrosin und D-Metionin sowie D-Leucin und D-Tyrosin und D-Tryptophan haben sich erfindungsgemäß besonders bewährt. Erfindungsgemäße Mund und Zahnpflege- und -reinigungsmittel, die sowohl D-Leucin als auch D-Tyrosin als auch D-Metionin enthalten, sind ebenso bevorzugt wie erfindungsgemäße Mund und Zahnpflege- und - reinigungsmittel, die sowohl D-Leucin als auch D-Tyrosin als auch D-Tryptophan enthalten.

Ganz besonders bevorzugte erfindungsgemäße Mund und Zahnpflege- und -reinigungsmittel enthalten sowohl D-Leucin als auch D-Tyrosin als auch D-Metionin als auch D-Tryptophan.

Als zweiten wesentlichen Inhaltsstoff enthalten die erfindungsgemäßen Mittel 0,1 bis 5 Gew.-% mindestens eines Antiplaque-Wirkstoffes aus den Gruppen der Alkypyridiniumsalze und/oder der Biguanide und/oder der Salze von und/oder p-Hydroxybenzoesäuremethyl-, -ethyl- oder propylester und/oder Natriumsorbat und/oder Natriumbenzoat.

Erfindungsgemäß bevorzugte Mund- und Zahnpflege- und -reinigungsmittel sind **dadurch gekennzeichnet, daß** sie Alkypyridiniumsalze, insbesondere Cetylpyridiniumchlorid enthalten. Weiter sind auch Biguanide z. B. Chlorhexidin und Thymol besonders geeignet.

Besonders geeignet sind darüber hinaus Organo-polyphosphonsäuren. Unter diesen Verbindungen hat sich insbesondere die Azacycloheptan-2,2-diphosphonsäure besonders bewährt, wobei auch die betreffenden Salze eingesetzt werden können. Erfindungsgemäß bevorzugte Mund und Zahnpflege- und -reinigungsmittel sind **dadurch gekennzeichnet, daß** sie 0,15 bis 4 Gew.-%, vorzugsweise 0,25 bis 3 Gew.-%, weiter bevorzugt 0,5 bis 2 Gew.-%, noch weiter bevorzugt 0,75 bis 1,5 Gew.-% und insbesondere 1 bis 1,25 Gew.-% Azacycloheptan-2,2-diphosphonsäure und/oder eins ihrer Salze enthalten.

Weitere bevorzugte erfindungsgemäße Mund und Zahnpflege- und -reinigungsmittel enthalten 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumsorbat.

Auch Natriumbenzoat kann erfindungsgemäß eingesetzt werden und weist in Kombination mit D-Aminosäuren eine gesteigerte Wirksamkeit gegen Plaque auf. Erfindungsgemäß ebenfalls bevorzugte Mund und Zahnpflege- und -reinigungsmittel enthalten 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumbenzoat.

Zusammenfassend enthalten besonders bevorzugte erfindungsgemäße Mittel
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,15 bis 4 Gew.-%, vorzugsweise 0,25 bis 3 Gew.-%, weiter bevorzugt 0,5 bis 2 Gew.-%, noch weiter bevorzugt 0,75 bis 1,5 Gew.-% und insbesondere 1 bis 1,25 Gew.-% Azacycloheptan-2,2-diphosphonsäure
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,15 bis 4 Gew.-%, vorzugsweise 0,25 bis 3 Gew.-%, weiter bevorzugt 0,5 bis 2 Gew.-%, noch weiter bevorzugt 0,75 bis 1,5 Gew.-% und insbesondere 1 bis 1,25 Gew.-% Azacycloheptan-2,2-diphosphonsäure
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin und 0,15 bis 4 Gew.-%, vorzugsweise 0,25 bis 3 Gew.-%, weiter bevorzugt 0,5 bis 2 Gew.-%, noch weiter bevorzugt 0,75 bis 1,5 Gew.-% und insbesondere 1 bis 1,25 Gew.-% Azacycloheptan-2,2-diphosphonsäure
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan und 0,15 bis 4 Gew.-%, vorzugsweise 0,25 bis 3 Gew.-%, weiter bevorzugt 0,5 bis 2 Gew.-%, noch weiter bevorzugt 0,75 bis 1,5 Gew.-% und insbesondere 1 bis 1,25 Gew.-% Azacycloheptan-2,2-diphosphonsäure
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,15 bis 4 Gew.-%, vorzugsweise 0,25 bis 3 Gew.-%, weiter bevorzugt 0,5 bis 2 Gew.-%, noch weiter bevorzugt 0,75 bis 1,5 Gew.-% und insbesondere 1 bis 1,25 Gew.-% Azacycloheptan-2,2-diphosphonsäure
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin und 0,15 bis 4 Gew.-%, vorzugsweise 0,25 bis 3 Gew.-%, weiter bevorzugt 0,5 bis 2 Gew.-%, noch weiter bevorzugt 0,75 bis 1,5 Gew.-% und insbesondere 1 bis 1,25 Gew.-% Azacycloheptan-2,2-diphosphonsäure
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan und 0,15 bis 4 Gew.-%, vorzugsweise 0,25 bis 3 Gew.-%, weiter bevorzugt 0,5 bis 2 Gew.-%, noch weiter bevorzugt 0,75 bis 1,5 Gew.-% und insbesondere 1 bis 1,25 Gew.-% Azacycloheptan-2,2-diphosphonsäure
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin und 0,15 bis 4 Gew.-%, vorzugsweise 0,25 bis 3 Gew.-%, weiter bevorzugt 0,5 bis 2 Gew.-%, noch weiter bevorzugt 0,75 bis 1,5 Gew.-% und insbesondere 1 bis 1,25 Gew.-% Azacycloheptan-2,2-diphosphonsäure
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan und 0,15 bis 4 Gew.-%, vorzugsweise 0,25 bis 3 Gew.-%, weiter bevorzugt 0,5 bis 2 Gew.-%, noch weiter bevorzugt 0,75 bis 1,5 Gew.-% und insbesondere 1 bis 1,25 Gew.-% Azacycloheptan-2,2-diphosphonsäure
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin und 0,15 bis 4 Gew.-%, vorzugsweise 0,25 bis 3 Gew.-%, weiter bevorzugt 0,5 bis 2 Gew.-%, noch weiter bevorzugt 0,75 bis 1,5 Gew.-% und insbesondere 1 bis 1,25 Gew.-% Azacycloheptan-2,2-diphosphonsäure
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan und 0,15 bis 4 Gew.-%, vorzugsweise 0,25 bis 3 Gew.-%, weiter bevorzugt 0,5 bis 2 Gew.-%, noch weiter bevorzugt 0,75 bis 1,5 Gew.-% und insbesondere 1 bis 1,25 Gew.-% Azacycloheptan-2,2-diphosphonsäure
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan und 0,15 bis 4 Gew.-%, vorzugsweise 0,25 bis 3 Gew.-%, weiter bevorzugt 0,5 bis 2 Gew.-%, noch weiter bevorzugt 0,75 bis 1,5 Gew.-% und insbesondere 1 bis 1,25 Gew.-% Azacycloheptan-2,2-diphosphonsäure
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan und 0,15 bis 4 Gew.-%, vorzugsweise 0,25 bis 3 Gew.-%, weiter bevorzugt 0,5 bis 2 Gew.-%, noch weiter bevorzugt 0,75 bis 1,5 Gew.-% und insbesondere 1 bis 1,25 Gew.-% Azacycloheptan-2,2-diphosphonsäure
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumsorbat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumsorbat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumsorbat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumsorbat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumsorbat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumsorbat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumsorbat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumsorbat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumsorbat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumsorbat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumsorbat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumsorbat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumsorbat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumbenzoat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumbenzoat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumbenzoat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumbenzoat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumbenzoat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumbenzoat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumbenzoat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumbenzoat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumbenzoat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumbenzoat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumbenzoat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumbenzoat
- 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin und 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan und 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumbenzoat

Die erfindungsgemäßen Zusammensetzungen können weitere Inhaltsstoffe enthalten:

Die erfindungsgemäßen Zusammensetzungen enthalten vorzugsweise mindestens ein Poliermittel. Als Poliermittel eignen sich prinzipiell alle für Zahnpasten bekannten Reibkörper, insbesondere solche, die keine Calciumionen enthalten. Bevorzugt geeignete Poliermittel-komponenten sind daher Kieselsäuren, Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% - bezogen auf die Gesamtzusammensetzung - enthalten sein.

Der Gesamtgehalt an Poliermitteln liegt vorzugsweise im Bereich von 5-50 Gew.-% des Zahnpflegemittels. Erfindungsgemäß bevorzugte Mittel enthalten Poliermittel innerhalb engerer Mengenbereiche. Hier sind erfindungsgemäß bevorzugte Mund- und Zahnpflege- und - reinigungsmittel **dadurch gekennzeichnet, daß** sie 1 bis 25 Gew.-%, vorzugsweise 2,5 bis 20 Gew.-%, weiter bevorzugt 5 bis 18 Gew.-% und insbesondere 7,5 bis 16 Gew.-% Poliermittel enthalten.

Besonders bevorzugt sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die so genannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m²/g, einer Partikelgröße von 0,5-20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10-20 Gew.-% der Zahnpaste. Bevorzugt geeignete Fällungskieselsäuren dieser Art weisen außerdem gerundete Ecken und Kanten auf und sind unter der Handelsbezeichnung Sident^{®}12 DS (DEGUSSA) erhältlich.

Andere Fällungskieselsäuren dieser Art sind Sident^{®} 8 (DEGUSSA) und Sorbosil^{®} AC 39 (Crosfield Chemicals). Diese Kieselsäuren zeichnen sich durch eine geringere Verdickungswirkung und eine etwas höhere mittlere Teilchengröße von 8 --14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pa.s aufweisen.

Zahnpasten, die eine deutlich höhere Viskosität von mehr als 100 Pa.s (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, so genannte Verdickungs-Kieselsäuren mit einer BET-Oberfläche von 150 -250 m²/g zu, z.B. die Handelsprodukte Sipernat 22 LS oder Sipernat^{®} 320 DS.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Erfindungsgemäß besonders bevorzugte Mund und Zahnpflege- und -reinigungsmittel enthalten ausschließlich Poliermittel aus der Gruppe der Kieselsäuren, Aluminiumhydroxid, Aluminiumoxid, Dicalciumphosphat-dihydrat (CaHPO₄ ·2H₂O) oder Gemische dieser Reibkörper. Diese Poliermittel haben ich als besonders effizient bei der Lösung der erfindungsgemäßen Aufgabe erwiesen. Ganz besonders bevorzugt enthalten die erfindungsgemäßen Zusammensetzungen bezogen auf ihr Gewicht 1 bis 30 Gew.-% Fällungskieselsäure(n) mit einer spezifischen Oberfläche nach ISO 5794-1, Anhang D von ≤ 55 m²/g. Vorzugsweise werden die Fällungskieselsäuren, die entsprechende spezifische Oberflächen aufweisen, innerhalb engerer Mengenbereiche eingesetzt, und insbesondere bevorzugt werden Fällungskiselsäuren eingesetzt, die noch niedrigere spezifische Oberflächen nach ISO 5794-1, Anhang D aufweisen. Bevorzugte erfindungsgemäße Mund- und Zahnpflege- und -reinigungsmittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 13,0 Gew.-% Fällungskieselsäure(n) mit einer spezifischen Oberfläche nach ISO 5794-1, Anhang D von ≤ 55 m²/g.

Besonders bevorzugte erfinddungsgemäße Mund und Zahnpflege- und -reinigungsmittel sind **dadurch gekennzeichnet, daß** sämtliche im Mittel enthaltenen Fällungskieselsäure(n) eine spezifische Oberfläche nach ISO 5794-1, Anhang D von ≤ 53 m²/g, vorzugsweise von ≤ 51 m²/g, weiter bevorzugt von von ≤ 49 m²/g und insbesondere von ≤ 47 m²/g aufweisen.

In weiter bevorzugten erfindungsgemäßen Mitteln sind die eingesetzten Fällungskieselsäuren durch weitere physikalische Parameter gekennzeichnet. Vorzugsweise einzusetzende Fällungskieselsäuren weisen Stampfdichten > 360 g/l (gemessen nach ISO 787-11), besonders bevorzugt > 375 g/l, weiter bevorzugt > 400 g/l und insbesondere > 425 g/l auf.

Es ist weiter bevorzugt, Fällungskieselsäuren einzusetzen, die eine DBP-Absorption gemäß DIN 53601 von weniger als 140 g/100 g aufweisen. Ganz besonders bevorzugte erfindungsgemäß einzusetzende Fällungskieselsäuren weisen eine DBP-Absorption gemäß DIN 53601 von weniger als 135 g/100 g, bevorzugt von eine DBP-Absorption gemäß DIN 53601 von weniger als 130 g/100 g und insbesondere von weniger als 125 g/100 g auf.

Erfindungsgemäß besonders bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer Stampfdichte (gemessen nach ISO 787-11), von > 425 g/l.

Erfindungsgemäß weiter bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer DBP-Absorption gemäß DIN 53601 von weniger als 125 g/100 g.

Erfindungsgemäß insbesondere bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer Stampfdichte (gemessen nach ISO 787-11), von > 425 g/l und einer DBP-Absorption gemäß DIN 53601 von weniger als 125 g/100 g.

Die erfindungsgemäßen Zusammensetzungen enthalten mindestens ein Poliermittel. Als Poliermittel eignen sich prinzipiell alle für Zahnpasten bekannten Reibkörper, insbesondere solche, die keine Calciumionen enthalten. Bevorzugt geeignete Poliermittel-komponenten sind daher Kieselsäuren, Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% - bezogen auf die Gesamtzusammensetzung - enthalten sein.

Der Gesamtgehalt an Poliermitteln liegt vorzugsweise im Bereich von 5 - 50 Gew.-% des Zahnpflegemittels. Erfindungsgemäß bevorzugte Mittel enthalten Poliermittel innerhalb engerer Mengenbereiche. Hier sind erfindungsgemäß bevorzugte Mund- und Zahnpflege- und - reinigungsmittel **dadurch gekennzeichnet, daß** sie 1 bis 25 Gew.-%, vorzugsweise 2,5 bis 20 Gew.-%, weiter bevorzugt 5 bis 18 Gew.-% und insbesondere 7,5 bis 16 Gew.-% Poliermittel enthalten.

Besonders bevorzugt sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die so genannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30- 60 Pa.s (20°C) in einer Menge von 10-20 Gew.-% der Zahnpaste. Bevorzugt geeignete Fällungskieselsäuren dieser Art weisen außerdem gerundete Ecken und Kanten auf und sind unter der Handelsbezeichnung Sident^{®}12 DS (DEGUSSA) erhältlich.

Andere Fällungskieselsäuren dieser Art sind Sident^{®} 8 (DEGUSSA) und Sorbosil^{®} AC 39 (Crosfield Chemicals). Diese Kieselsäuren zeichnen sich durch eine geringere Verdickungswirkung und eine etwas höhere mittlere Teilchengröße von 8 --14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pa.s aufweisen.

Zahnpasten, die eine deutlich höhere Viskosität von mehr als 100 Pa.s (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, so genannte Verdickungs-Kieselsäuren mit einer BET-Oberfläche von 150 -250 m²/g zu, z.B. die Handelsprodukte Sipernat 22 LS oder Sipernat^{®} 320 DS.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Erfindungsgemäß besonders bevorzugte Mund und Zahnpflege- und -reinigungsmittel enthalten ausschließlich Poliermittel aus der Gruppe der Kieselsäuren, Aluminiumhydroxid, Aluminiumoxid, Dicalciumphosphat-dihydrat (CaHPO₄ ·2H₂O) oder Gemische dieser Reibkörper. Diese Poliermittel haben ich als besonders effizient bei der Lösung der erfindungsgemäßen Aufgabe erwiesen. Ganz besonders bevorzugt enthalten die erfindungsgemäßen Zusammensetzungen bezogen auf ihr Gewicht 1 bis 30 Gew.-% Fällungskieselsäure(n) mit einer spezifischen Oberfläche nach ISO 5794-1, Anhang D von ≤ 55 m²/g. Vorzugsweise werden die Fällungskieselsäuren, die entsprechende spezifische Oberflächen aufweisen, innerhalb engerer Mengenbereiche eingesetzt, und insbesondere bevorzugt werden Fällungskieselsäuren eingesetzt, die noch niedrigere spezifische Oberflächen nach ISO 5794-1, Anhang D aufweisen. Bevorzugte erfindungsgemäße Mund- und Zahnpflege- und -reinigungsmittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 13,0 Gew.-% Fällungskieselsäure(n) mit einer spezifischen Oberfläche nach ISO 5794-1, Anhang D von ≤ 55 m²/g.

Besonders bevorzugte erfinddungsgemäße Mund und Zahnpflege- und -reinigungsmittel sind **dadurch gekennzeichnet, daß** sämtliche im Mittel enthaltenen Fällungskieselsäure(n) eine spezifische Oberfläche nach ISO 5794-1, Anhang D von ≤ 53 m²/g, vorzugsweise von ≤ 51 m²/g, weiter bevorzugt von von ≤ 49 m²/g und insbesondere von ≤ 47 m²/g aufweisen.

In weiter bevorzugten erfindungsgemäßen Mitteln sind die eingesetzten Fällungskieselsäuren durch weitere physikalische Parameter gekennzeichnet. Vorzugsweise einzusetzende Fällungskieselsäuren weisen Stampfdichten > 360 g/l (gemessen nach ISO 787-11), besonders bevorzugt > 375 g/l, weiter bevorzugt > 400 g/l und insbesondere > 425 g/l auf.

Es ist weiter bevorzugt, Fällungskieselsäuren einzusetzen, die eine DBP-Absorption gemäß DIN 53601 von weniger als 140 g/100 g aufweisen. Ganz besonders bevorzugte erfindungsgemäß einzusetzende Fällungskieselsäuren weisen eine DBP-Absorption gemäß DIN 53601 von weniger als 135 g/100 g, bevorzugt von eine DBP-Absorption gemäß DIN 53601 von weniger als 130 g/100 g und insbesondere von weniger als 125 g/100 g auf.

Erfindungsgemäß besonders bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer Stampfdichte (gemessen nach ISO 787-11), von > 425 g/l.

Erfindungsgemäß weiter bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer DBP-Absorption gemäß DIN 53601 von weniger als 125 g/100 g.

Erfindungsgemäß insbesondere bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer Stampfdichte (gemessen nach ISO 787-11), von > 425 g/l und einer DBP-Absorption gemäß DIN 53601 von weniger als 125 g/100 g.

Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P ₂O₇, Na₂K₂P₂O ₇, Na₂H₂P₂O₇ und K ₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Erfindungsgemäß bevorzugte Mund- und Zahnpflege- und -reinigungsmittel sind **dadurch gekennzeichnet, daß** sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Die erfindungsgemäßen Mittel, insbesondere die Zahnpasten, können auch Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Mund und Zahnpflege- und -reinigungsmittel können darüber hinaus mit besonderem Vorzug Antikaries-Wirkstoffe enthalten. Diese können beispielsweise aus organischen oder anorganischen Fluoriden ausgewählt sein, z. B. aus Natriumfluorid, Kaliumfluorid, Natriummonofluorphosphat und Natriumfluorosilikat. Auch Zinkfluorid, Zinn-(II)-fluorid sind bevorzugt. Bevorzugt sollte eine Menge von 0,01 - 0,2 Gew.-% Fluor in Form der genannten Verbindungen enthalten sein.

Erfindungsgemäße Mund- und Zahnpflege- und -reinigungsmittel, die zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise von 0,1 bis 2,5 Gew.% und insbesondere von 0,2 bis 1,1 Gew.%, jeweils bezogen auf das gesamte Mittel, enthalten, sind erfindungsgemäß bevorzugt.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose sowie Maltose und Dextrose.

Ein weiterer Gegenstand der vorliegenden Erfindung ist die Verwendung von erfindungsgemäßen Mitteln zur Reinigung von Zähnen mittels Zahnbürsten.

Ein weiterer Gegenstand der vorliegenden Erfindung ist ein Verfahren zum Reinigen von Zähnen, bei dem ein erfindungsgemäßes Mittel auf den Bürstenkopf einer Zahnbürste aufgetragen und mit der Zahnbürste die Zähne geputzt werden.

Bezüglich bevorzugter Ausführungsformen der erfindungsgemäßen Verwendung und der erfindungsgemäßen Verfahren gilt mutatis mutandis das zu den erfindungsgemäßen Mitteln Gesagte.

## Patentansprüche

1. Mund- und Zahnpflege- und -reinigungsmittel, enthaltend - bezogen auf sein Gewicht-
a) 0,01 bis 1 Gew.-% mindestens einer D-Aminosäure,
b) 0,1 bis 5 Gew.-% mindestens eines Antiplaque-Wirkstoffes aus den Gruppen
a. der Alkypyridiniumsalze und/oder
b. der Biguanide und/oder
c. der Salze von Organo-polyphosphonsäuren und/oder
d. p-Hydroxybenzoesäuremethyl-, -ethyl- oder propylester und/oder
e. Natriumsorbat und/oder
f. Natriumbenzoat.

2. Mund- und Zahnpflege- und -reinigungsmittel nach Anspruch 1, **dadurch gekennzeichnet, daß** es 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Leucin enthält.

3. Mund und Zahnpflege- und -reinigungsmittel nach einem der Ansprüche 1 oder 2, **dadurch gekennzeichnet, daß** es es 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tyrosin enthält

4. Mund und Zahnpflege- und -reinigungsmittel nach einem der Ansprüche 1 bis 3, **dadurch gekennzeichnet, daß** es 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Metionin enthält.

5. Mund und Zahnpflege- und -reinigungsmittel nach einem der Ansprüche 1 bis 4, **dadurch gekennzeichnet, daß** es 0,015 bis 0,75 Gew.-%, vorzugsweise 0,02 bis 0,5 Gew.-%, weiter bevorzugt 0,05 bis 0,4 Gew.-%, noch weiter bevorzugt 0,075 bis 0,3 Gew.-% und insbesondere 0,1 bis 0,25 Gew.-% D-Tryptophan enthält.

6. Mund und Zahnpflege- und -reinigungsmittel nach einem der Ansprüche 1 bis 5, **dadurch gekennzeichnet, daß** es sowohl D-Leucin als auch D-Tyrosin enthält.

7. Mund und Zahnpflege- und -reinigungsmittel nach einem der Ansprüche 1 bis 6, **dadurch gekennzeichnet, daß** es 0,15 bis 4 Gew.-%, vorzugsweise 0,25 bis 3 Gew.-%, weiter bevorzugt 0,5 bis 2 Gew.-%, noch weiter bevorzugt 0,75 bis 1,5 Gew.-% und insbesondere 1 bis 1,25 Gew.-% Azacycloheptan-2,2-diphosphonsäure und/oder eins ihrer Salze enthält.

8. Mund und Zahnpflege- und -reinigungsmittel nach einem der Ansprüche 1 bis 7, **dadurch gekennzeichnet, daß** es 0,125 bis 4 Gew.-%, vorzugsweise 0,15 bis 3 Gew.-%, weiter bevorzugt 0,15 bis 2 Gew.-%, noch weiter bevorzugt 0,2 bis 1 Gew.-% und insbesondere 0,3 bis 0,6 Gew.-% Natriumbenzoat enthält.

9. Verwendung von Mitteln nach einem der Ansprüche 1 bis 8 zur Reinigung von Zähnen mittels Zahnbürsten.

10. Verfahren zum Reinigen von Zähnen, **dadurch gekennzeichnet, daß** ein Mittel nach einem der Ansprüche 1 bis 7 auf den Bürstenkopf einer Zahnbürste aufgetragen und mit der Zahnbürste die Zähne geputzt werden.
